# EUROPEAN PATENT SPECIFICATION

(11) **EP 1 216 017 B1**
(45) Date of publication and mention of the grant of the patent: **12.09.2007**
(21) Application number: 00964267.9
(22) Date of filing: 29.09.2000
(51) Int. Cl.: A61K 8/67, A61K 8/97, A61Q 5/08

(54) **HAIR CARE COMPOSITION**
HAARPFLEGEMITTEL
COMPOSITION DE SOIN CAPILLAIRE

(30) Priority: 30.09.1999 GB 9923023
(43) Date of publication of application: 26.06.2002
(73) Proprietor: The Boots Company PLC, Nottingham, Nottinghamshire NG2 3AA (GB)
(72) Inventor: GALLEY, Edward, Nottingham Nottinghamshire NG2 3AA (GB); HANRAHAN, Brian, Nottingham Nottinghamshire NG2 3AA (GB)
(74) Representative: Jones, Stephen Anthony
(86) International application number: PCT/EP2000/009648
(87) International publication number: WO 2001/022924

(56) References cited:
- EP-A- 0 894 494
- EP-A- 1 025 835
- WO-A-99/66881
- DATABASE WPI Week 199824 Derwent Publications Ltd., London, GB; AN 1998-266985 XP002156768 & JP 10 087440 A (KANEBO)
- DATABASE CAPLUS [Online] retrieved from STN Database accession no. 1998:214544 XP002156767 & JP 10 088193 A (KANEBO) 7 April 1998 (1998-04-07)
- PATENT ABSTRACTS OF JAPAN vol. 014, no. 485 (C-0772) & JP 02 200610 A (NICHIREI CORP.)

## Description

The present invention relates to a method for removing hair colour and/or imparting shine and condition to the hair.

Beautiful hair is seen as very attractive. Many people to improve their appearance, dye their hair, either to give it a more attractive colour or to hide grey hairs. Accordingly there is a huge market in permanent hair dyes.

Hair dyeing does not always achieve the desired effect. Sometimes people dislike the colour after they have applied it, find it unflattering or unsuitable, or just grow bored of it. Many times this is because the shade shown on the container is not representative of the final colour of hair on the scalp. Although the general colour of the hair is determined by the components of the dye, the specific shade will vary according to the base colour of the users hair. The hair colourants will react differently accordingly. Thus sometimes the result will be too dark and unnatural looking or unwanted tones will appear.

In the case of a hair dyeing disaster the options are fairly sparse. Hair dyes that are intended to be permanent will not be easily removed. Harsh chemical processes are required to break the bonds that hold the dye to the hair. This tends to damage the hair leaving it dry, weak and in bad condition. However the alternative is to leave the hair to grow out, removing the dye naturally. Another option is to use a product containing lemon juice.

Lemon juice is well known for its use in bleaching/removing hair colour from hair. These properties have been ascribed to the ascorbic acid which is present in lemon juice. Compositions containing lemon juice have also been found to impart shine and condition to the hair. There are a number of products commercially available containing lemon juice or ascorbic acid designed to lighten hair colour or to provide the hair with shine and condition.

The present invention provides a method for removing hair colour and/or for imparting shine and condition to the hair involving the application of defined haircare compositions comprising acerola cherry extract. The acerola cherry is commonly known as the Barbados or West Indian cherry, Cereza, Cerisier or Semeruco. Its scientific name is *Malpighia punicfolia L.* The bright red fruit that these trees produce are very high in vitamin C, on average 1500mg/100g of fresh fruit, though amounts of up to 4000mg/100g are not uncommon. The fruits are sometimes used as a source of vitamin C for nutritional supplements.

The acerola cherry extract may be obtained as a dry powder extract by a process in which the fruit is washed and mashed using suitable bladed or auger processor. The mash is then coarsely filtered to remove the fruit stone. The resulting liquor is then filtered using a bag filter press to produce a low viscosity liquid free from extraneous matter. The filtrate is then dried to produce a fine powder using a spray dryer or a freeze dryer and mill. Alternatively the acerola cherry extract may be obtained as a liquid extract by a process in which the fruit is mashed and filtered as above to produce a low viscosity liquid. This is then mixed with equal volume of a glycol (glycerin, propylene glycol, butylene glycol) and evaporated using a thin film evaporator to achieve a water availability value of zero to produce a stable liquid.

Compositions containing acerola cherry extract have been found to be extremely efficacious at removing hair colour, far more than would be expected in view of the level of ascorbic acid. The compositions also impart shine and condition to the hair. Lemon juice which has traditionally been used to lighten hair colour is not really very effective. It has to be repeatedly applied and the treated hair preferably exposed to sunlight. Further, lemon juice is not capable of being stored in products with a long shelf life. Ascorbic acid is a more limited ingredient. It is believed that many of the other components of lemon juice assist ascorbic acid in providing the above effects. Acerola cherry extract is not only stable, but is required in much smaller quantities than either lemon juice or ascorbic acid.

Without being bound by any theory it is believed that acerola cherry extract contains a wealth of additional compounds which when combined with the ascorbic acid present, produce a synergistic combination. Thus small amounts of the acerola cherry extract containing relatively small amounts of ascorbic acid is capable of having a disproportionate cosmetic effect on hair.

Suitable sources of acerola cherry extract include "Acerola Juice Powder" from Gee Lawson. The product is supplied as a dry flaky powder. In this document all weights and percentages refer to the amount of acerola cherry extract as dry powder as a percentage of the total amount of any such composition in its ready-to-use form.

One aspect of the present invention provides a method for removing hair colour and/or for imparting shine and condition to the hair involving providing acerola cherry extract in dry powder form, dissolving said dry powder in water to form a haircare composition as defined herein and applying said composition to the hair. Compositions useful in the invention may contain between 0.01 and 15.0% w/w of the acerola cherry extract, preferably contain between 0.1 and 3.0% w/w of the acerola cherry extract and most preferably about 0.15% w/w of the acerola cherry extract.

The compositions useful in accordance with the present invention may contain additional ascorbic acid or a derivative thereof. Suitable derivatives of ascorbic acid include ascorbyl salts (for example the sodium ascorbate or magnesium ascorbate (Vitacedone* UCIB), ascorbyl esters (for example ascorbyl palmitate), magnesium ascorbyl phosphate (Nikkol VC-PMG*, Jan Dekker), disodum ascorbyl sulphate (Nikkol VC-SS*, Jan Dekker), ascorbic acid polypeptide (Vitazyme C*, Brooks) or ascorbylmethylsilanol pectinate (Ascorbilane*,Exsymol)

The method of the present invention thus also involves haircare compositions containing acerola cherry extract and additional ascorbic acid or a derivative thereof. Such compositions are suitable for both removing hair colour and imparting condition and shine to the hair. Such compositions may contain between 0.01 and 15.0% w/w , preferably between 0.1 and 3.0% w/w, more preferably about 0.15% w/w of the acerola cherry extract and between 0.1 and 20.0% w/w of the additional ascorbic acid or a derivative thereof. When the haircare compositions are intended for removing hair colour the amount of additional ascorbic acid or a derivative thereof is preferably in the range 5 to 10% w/w. When the haircare compositions are intended for imparting shine and condition to the hair the amount of additional ascorbic acid or a derivative thereof is preferably between 2.0 and 5.0% w/w.

The ranges of the components refer to the amounts of material present in a composition ready for use. A preferred composition form is a dry powder, capable of being dissolved in water to give the composition ready to use.

The term "hair care composition" as used herein includes so-called "hot oil" treatments, shampoos, conditioners, powders, hair dyes, mousses, foams, gels, creams, waxes, masks, muds, styling sprays, lotions and rinses, all suitable for use on animals, preferably on humans, most preferably on the human head.

The hair care compositions of the present invention may also include other additives known by those skilled in the art to be suitable for inclusion in a hair care compositions. These may include;

For example, preservatives may be added to the composition such as 2-bromo-2-nitropropane-1,3-diol (bronopol, which is available commercially under the trade name Myacide RTM), benzyl alcohol, diazolidinyl urea, imidazolidinyl urea, methyl paraben, phenoxy ethanol, propyl paraben, sodium methyl paraben and sodium propyl paraben, suitably in an amount of from about 0.01 % to about 10% by weight of the composition.

Thickeners and viscosity modifying agents may be added to the composition, such as amine oxides, block polymers of ethylene oxide and propylene oxide (for example, those available from BASF Wyandotte under the trade name "Pluronic" RTM), ethoxylated fatty alcohols, cellulosic derivatives such as hydroxypropylmethyl cellulose, salt (NaCl), phthalic acid amide, polyvinyl alcohols and fatty alcohols, suitably in an amount of from about 0.5% to about 10% by weight of the composition. The composition may also include gelling agents such as PVM, MA, or a decadiene crosspolymer (available under the trade name Stabilez 06), suitably in an amount of from about 0.1 % to about 2% by weight of the composition.

Sequestering agents may be added to the composition, such as ethylenediamine tetraacetic acid and salts thereof, suitably in an amount of from about 0.005% to about 0.5% by weight of the composition.

The composition may also include resins such as: octylacrylamide / acrylates / butylaminomethacrylate copolymer (available under the trade name Amphomer RTM); ethyl ester of polyvinylmethyl (hereinafter known as PVM) / methylacrylate (hereinafter known as MA) copolymer (available under the trade name Ultrahold 8A RTM); vinyl acetate (hereinafter known as VA) / crotonates / vinyl neodecanate copolymer (available under the trade name Adhesive 28-2930 NAL); acrylates / acrylamide copolymer (available under the trade name Gantrez ES225 RTM); vinyl acetate / crotonic acid / vinyl propionate copolymer (available under the trade name Luviset CAP RTM); polyvinylpropionate (hereinafter known as PVP) / VA / vinylpropionate copolymer (available under the trade name Laviskol VAP RTM); octylacrylamide / acrylate copolymer (available under the trade names Versatyl 90 RTM or Lovocryl 47 RTM); vinyl caprolactam / PVP / dimethylaminoethyl methacrylate copolymer (available under the trade name (H₂O LD EP-1); PVM / MA copolymer (available under the trade name Gantrez RTM); and vinyl acetate / butyl maleate / isobornyl acrylate copolymer (available under the trade name Advantage CP RTM). These resins may be present suitably in an amount of from about 0.1% to about 10% by weight of the composition.

The composition may also include slip aids such as phenyl trimethicone, suitably in an amount of from about 0.1% to about 10% by weight of the composition.

The composition may also include vitamins such as biotin, suitably in an amount of from about 0.01 % to about 1.0% by weight of the composition.

The composition may also include waxes such as cocoa butter suitably in an amount of from about 1% to about 99% by weight of the composition.

The composition may also comprise suitable, cosmetically acceptable diluents, carriers and/or propellants such as dimethyl ether.

The composition may also include pearlising agents such as stearic monoethanolamide, suitably in an amount of from about 0.01 % to about 10% by weight of the composition.

Perfumes may be added suitably in an amount of from about 0.01 % to about 2% by weight of the composition, as may water soluble dyes such as tartrazine, suitably in an amount of from about a trace amount (such as 1 x 10⁻⁵ %) to about 0.1 % by weight of the composition.

The composition may also include pH adjusting agents such as sodium hydroxide, aminomethyl propanol, triethanolamine, suitably in an amount of from about 0.01 % to about 10% by weight of the composition.

The composition may be buffered by means well known in the art, for example by use of buffer systems comprising succinic acid, citric acid, lactic acid, and acceptable salts thereof, phosphoric acid, mono- or disodium phosphate and sodium carbonate. Suitably, the composition may have a pH between about 3 and about 10, preferably between about 4 and about 8.

The composition may also include an antidandruff agent such as salicylic acid or zinc pyrithione or octopyrox suitably in an amount of from about 0.1% to about 5% by weight of the composition.

Surfactants may be included, such as cosmetically acceptable salts of alkyl ether sulphates, alkyl and alkylamidoalkyl betaines, ethoxylated alcohols, polyethyleneglycol carboxylates, acceptable salts of alkyl sulphates (such as ammonium lauryl sulphate), acceptable salts of alkyl ether sulphates (such as ammonium laureth sulphate of sodium laureth sulhate), sulphosuccinates (such as disodium laureth sulphosuccinate), amphoacetates and amphodiacetates (such as disodium cocoamphodiacetate), alkylpolyglucosides and alcohol sulphonates.

Broadly in accordance with a still further aspect of the present invention there is provided a method of treating hair (for example washing, conditioning and/or styling hair) by application of a composition as described herein.

The invention will be understood with reference to the non-limiting tests and formulation examples described hereinafter. The amounts of the components present are given on a weight/weight or a weight/volume basis as indicated in each example.

### Formulation Example 1 - Pearlised Shampoo

| Component | % w/v |
|---|---|
| Sodium laureth-2 sulphate (70%) in water | 6.38 |
| Mixture of cocoamidopropyl betaine (30%), NaCl (5%) and water | 2.85 |
| Polyquaternium-39 (10%) in water | 0.50 |
| Mixture of sodium lauryl ether sulphate (20%), ethylene glycol distearate (20%), cocoyl monoethanolamide (3.5%), laureth-10 (3.5%) and water | 0.85 |
| 2-Bromo-2-nitropropane-1,3-diol | 0.02 |
| Pure, vacuum-dried NaCl | 2.00 |
| Citric acid monohydrate | qs |
| Acerola cherry extract powder | 0.15 |
| Purified water | to 100 |

The pearlised shampoo is prepared as follows. The water is slowly added to the sodium laureth-2 sulphate which is stirred. Once the water is mixed in, the remaining ingredients (except the acerola cherry extract), are added to the water phase whilst the water phase is stirred. The pH of the resulting mixture is adjusted to between 5 to 6 and then the acerola cherry extract is added whilst the mixture is stirred.

### Formulation Example 2 - Conditioners

| Component | %w/v |
|---|---|
| Cetyl alcohol | 4.00 |
| Mixture of cetyl alcohol (80%) and polyethylene glycol (20) stearate (20%) | 2.00 |
| Hydroxyethyl cellulose | 1.00 |
| Polyquaternium-39 (10%) in water | 1.00 |
| Hydrolysed soya protein (20%) in water | 0.10 |
| Panthenol (75%) in water | 0.05 |
| 2-Bromo-2-nitropropane-1,3-diol | 0.02 |
| Citric acid | 0.02 |
| Acerola cherry extract | 0.15 |
| Purified water | to 100 |

The conditioners are prepared as follows. The hydroxyethyl cellulose is dispersed in water at 50°C to 60°CO using a high shear mixer. The pH of the resulting mixture is adjusted as required. The cetyl alcohol and the cetyl alcohol and polyethylene glycol (20) stearate mixture are heated to 60C and then added to the water phase whilst the water phase is mixed with a high shear mixer. The resulting mixture is cooled and the remaining ingredients are added.

### Formulation Example 3 - Shampoo

| Component | % w/v |
|---|---|
| Sodium laureth-2 sulphate (70%) in water | 6.38 |
| Mixture of cocoamidopropylbetaine (30%), salt | 3.84 |
| (5%) and water Polyquaternium-39 (10%) in water | 0.50 |
| 2-Bromo-2-nitropropane-1,3-diol | 0.02 |
| Pure, vacuum-dried NaCl | 1.50 |
| Citric acid monohydrate | 0.20 |
| Acerola cherry extract | 3.00 |
| Purified water | to 100 |

The shampoo is prepared by the method used to prepare formulation example 1.

### Formulation Example 4 - Leave-in Conditioner

| Component | %w/v |
|---|---|
| D-panthenol (75%) in water | 0.75 |
| Polyquatemium-39 (10%) in water | 10.00 |
| Dimethicone propyl PG betaine | 0.50 |
| Perfume | 0.10 |
| PEG-40 hydrogenated castor oil | 0.50 |
| Methylchloroisothiazoline, methylisothiazolinone, magnesium nitrate and water | 0.08 |
| Acerola cherry extract | 3.00 |
| Purified water | to 100 |

The conditioner is prepared as follows. The perfume and the PEG-40 hydrogenated castor oil are stirred together and then the rest of the ingredients are added to this mixture which is stirred.

### Formulation Example 5 - Non-Aerosol Volumising Gel Spray

| Component | % w/v |
|---|---|
| Denatured alcohol | 84.08 |
| Purified water | 8.34 |
| PVP / VA copolymer | 1.74 |
| PVP | 1.20 |
| Isopropyl alcohol | 0.60 |
| Rosin acrylate | 0.40 |
| Phenyl dimethicone | 0.30 |
| Panthenol | 0.30 |
| Benzophenone-4 | 0.02 |
| Polyquaternium-6 (40%) in water | 0.05 |
| Acerola cherry extract | 2.00 |

The volumising gel spray is prepared as follows. The PVP/VA copolymer is dissolved in a mixture of the denaturated alcohol and the isopropyl alcohol whilst the alcohols are being stirred for 30 minutes. The phenyl dimethicone and acerola cherry extract are added to this mixture. The benzophenone-4, polyquaterium-6, panthenol and PVP are added to the water which is stirred to dissolve these ingredients. The resultant aqueous solution is added to the alcohol mixture whilst the alcohol mixture is stirred.

### Formulation Example 6 - Non-Aerosol Hairspray (Firm Hold)

| Component | % w/v |
|---|---|
| Denaturated alcohol | to 100 |
| Vinyl acetate / crotonates / vinyl neodecanoate copolymer | 4.50 |
| Purified water BP | 3.62 |
| PVP / VA copolymer | 0.95 |
| Aminomethyl propanol | 0.40 |
| Panthenol | 0.30 |
| Rosin acrylate | 0.20 |
| Benzophenone-4 | 0.02 |
| Polyquaternium-6 (40%) in water | 0.05 |
| Phenyl dimethicone | 0.20 |
| Acerola cherry extract | 0.20 |

The hairspray is prepared as follows. The denaturated alcohol and aminomethyl propanol are stirred together. The remaining ingredients (except the vinyl acetate / crotonates / vinyl neodecanoate copolymer) are added to the ethanol mix and the resultant mixture is stirred. Finally the copolymer is added whilst the mixture is agitated.

### Formulation Example 7 - Reviving Hair Curls

| Component | % w/v |
|---|---|
| Denatured alcohol | 68.23 |
| Purified water (BP) | 27.43 |
| PEG-7 glyceryl cocoate | 0.40 |
| PVP | 0.40 |
| Polyquaternium-4 | 0.37 |
| Panthenol | 0.30 |
| Perfume | 0.10 |
| Tocopheryl acetate | 0.05 |
| Polyquaternium-39 (10%) in water | 0.50 |
| Benzophenone-4 | 0.02 |
| Methylparaben | 0.01 |
| Propylparaben | 0.00 |
| Acerola cherry extract | 1.00 |

The spray is prepared as follows. The polyquaternium-4 is stirred into the water for 30 minutes. The remaining ingredients (except the acerola cherry extract and denaturated alcohol) are added. Then the denaturated alcohol and acerola cherry extract are added to the bulk mixture.

### Formulation Example 8 - Mousse (Firm Hold)

| Component | % w/v |
|---|---|
| Propane | 3.85 |
| Butane | 2.10 |
| PVP / VA Copolymer | 1.59 |
| Isobutane | 1.05 |
| PVP | 0.93 |
| Polyquaternium-4 | 0.86 |
| Ceteth-20 | 0.47 |
| C12-13 Pareth-3 | 0.28 |
| Polyquaternium-11 | 0.19 |
| Perfume | qs |
| Panthenol | 0.14 |
| Polyquatemium-39 (10%) in water | 0.47 |
| Benzophenone-4 | 0.01 |
| Preservative | qs |
| Acerola cherry extract | 0.10 |
| Purified water BP | to 100 |

The mousse is prepared as follows. The polyquaterium-4 is added to water and the mixture stirred rapidly for 30 minutes. The acerola cherry extract is pre-dissolved in water and then added to the water mixture. The remaining solid or liquid ingredients are added to this mixture with stirring. The resulting mixture was then packed into pressurised containers and the gaceous propellants added.

### Formulation Example 9 - Hair Gel

| Component | % w/v |
|---|---|
| Purified water BP | to 100 |
| PVP | 3.50 |
| Carbomer | 1.00 |
| Aminomethyl propanol | 0.40 |
| Dimethicone copolyol | 0.20 |
| PG-2 ceteareth-9 | 0.20 |
| Panthenol | 0.08 |
| Disodium EDTA | 0.05 |
| Quaternium-15 | 0.05 |
| Polyquaternium-39 (10%) in water | 0.50 |
| Benzophenone-4 | 0.02 |
| Methylparaben | qs |
| Propylparaben | qs |
| Acerola cherry extract | 0.15 |

The hair gel is prepared as follows. The carbomer is dispersed in two thirds of the water with the disodium EDTA using a high shear mixer. The aminomethyl propanol is then added to this carbomer dispersion. The rest of the ingredients are added to the remaining water. This water phase mixture is then added to the carbomer dispersion.

### Formulation Example 10 - Dry Mousse

| Component | %w/v |
|---|---|
| Propane | 3.85 |
| Butane | 2.10 |
| PVP / VA Copolymer | 1.30 |
| Isobutane | 1.00 |
| PVP | 1.00 |
| Polyquaternium-4 | 1.00 |
| Ceteth-20 | 0.50 |
| C12-13 Pareth-3 | 0.30 |
| Polyquaternium-11 | 0.20 |
| Perfume | 0.20 |
| Propylene glycol | 0.05 |
| Polyquaternium-39 (10%) in water | 0.50 |
| Preservatives | qs |
| Acerola cherry extract | 0.30 |
| Purified water BP | to 100 |

The mousse is prepared by the method used to prepare formulation example 8.

### Formulation Example 11 - Hair Texturising Wax

| Component | % w/v |
|---|---|
| Petrolatum | to 100 |
| Hydrogenated coco-glycerides | 5.00 |
| Paraffin | 5.00 |
| Propylene glycol | 1.05 |
| PEG-60 hydrogenated castor oil | 1.00 |
| Purified water BP | 0.05 |
| Polyquaternium-39 (10%) in water | 0.05 |
| Acerola cherry extract | 0.10 |
| Preservatives | qs |

The hair texturising wax is prepared as follows. The propylene glycol and acerola cherry extract are stirred together. The PEG-60 hydrogenated castor oil and polyquaternium-39 are stirred into the acerola cherry extract. The remaining ingredients are melted together at 80°C, then cooled to 60°C and added to the acerola cherry extract mixture.

### Formulation Example 12 - Hair Finishing Spray

| Component | % w/v |
|---|---|
| Denatured alcohol | to 100 |
| Butane | 27.00 |
| Isobutane | 12.00 |
| Propane | 11.00 |
| Acrylates / acrylamide copolymer | 3.00 |
| Purified water BP | 2.00 |
| Aminomethyl propanol | 0.24 |
| Phenyl dimethicone | 0.20 |
| Polyquaternium-6 (40%) in water | 0.05 |
| Propylene glycol | 1.50 |
| Acerola cherry extract | 1.00 |

The finishing spray is prepared as follows. The alcohol and aminomethyl propanol are stirred together. The acrylates / acrylamide copolymer is slowly added to the alcohol mixture whilst the alcohol mixture is stirred. The phenyl dimethicone, propylene glycol and acerola cherry extract are stirred into the mixture and dissolved. The polyquaternium-6 and water are stirred together and added to the bulk mixture. The resulting mixture was then packed into pressurised containers and the gaseous propellants added.

### Formulation Example 13 - Finishing Spray (Firm Hold)

| Component | % w/v |
|---|---|
| Denatured alcohol | To 100 |
| Butane | 16.20 |
| Isobutane | 7.20 |
| Propane | 6.60 |
| VA / crotonates / vinyl propionate copolymer | 3.00 |
| Purified water BP | 2.00 |
| Aminomethyl propanol | 0.25 |
| Phenyl dimethicone | 0.10 |
| Polyquaternium-6 (40%) in water | 0.05 |
| Propylene glycol | 1.50 |
| Acerola cherry extract | 0.50 |

The finishing spray is prepared by an analogous method to that used to prepare formulation example 12 in which the VA / crotonates / vinyl propionate copolymer replaces the acrylate / acrylamide copolymer. The resulting mixture was then packed into pressurised containers and the gaseous propellants added.

### Formulation Example 14 - Pearlised Shampoo

| Component | % w/v |
|---|---|
| Sodium laureth-2 sulphate (27%) | 20.00 |
| Cocoamidopropyl betaine | 3.00 |
| Polyquaternium-39 (10%) in water | 0.50 |
| Sodium laureth, glycol distearate and cocoamide DEA | 2.00 |
| Viscosity adjuster | qs |
| PH adjuster | qs |
| Perfume | 0.40 |
| Preservative | qs |
| Acerola cherry extract | 0.05 |
| Purified water BP | to 100 |

The pearlised shampoo is prepared by the same method used to prepare formulation example 1.

### Formulation Example 15 - Shampoo

| Component | %w/v |
|---|---|
| Sodium laureth-2 sulphate (27%) | 20.00 |
| Cocoamidopropyl betaine | 4.00 |
| Polyquaternium-39 (10%) in water | 0.50 |
| Perfume | 0.40 |
| Preservative | qs |
| pH adjuster | qs |
| Viscosity adjuster | qs |
| Acerola cherry extract | 0.30 |
| Purified water BP | to 100 |

The shampoo is prepared by the same method used to prepare formulation Example 1.

### Formulation Example 16 - Conditioning Shampoo

| Component | %w/v |
|---|---|
| Sodium laureth-2 sulphate (27%) | 30.00 |
| Ammonium lauryl sulphate | 20.00 |
| Cocoamidopropyl betaine | 10.00 |
| Dimethicone propyl PG betaine | 0.50 |
| Guar hydroxypropyltrimonium chloride | 0.20 |
| Sodium laureth, glycol distearate and cocoamide DEA | 2.00 |
| Polyquaternium-39 (10%) in water | 2.50 |
| Perfume | qs |
| PH adjuster | qs |
| Preservatives | qs |
| Acerola cherry extract | 0.15 |
| Magnesium ascorbyl phosphate | 1.5 |
| Purified water BP | to 100 |

The conditioning shampoo is prepared by the same method used to prepare formulation example 1.

### Formulation Example 17 - Conditioning Shampoo

| Component | %w/v |
|---|---|
| Sodium laureth-2 sulphate (27%) | 30.00 |
| Ammonium lauryl sulphate | 20.00 |
| Cocoamidopropyl betaine | 10.00 |
| Dimethicone propyl PG betaine | 0.50 |
| Guar hydroxypropyltrimonium chloride | 0.20 |
| Sodium laureth, glycol distearate and cocoamide DEA | 2.00 |
| Polyquaternium-39 (10%) in water | 2.50 |
| Perfume | 0.40 |
| PH adjuster | qs |
| Preservatives | qs |
| Acerola cherry extract | 0.15 |
| Ascorbic acid | 1.5 |
| Purified water BP | to 100 |

The conditioning shampoo is prepared by the same method used to prepare formulation example 1.

### Formulation Example 18 - Conditioning Shampoo

| Component | %w/v |
|---|---|
| Sodium laureth-2 sulphate (27%) | 30.00 |
| Ammonium lauryl sulphate | 20.00 |
| Cocoamidopropyl betaine | 10.00 |
| Dimethicone propyl PG betaine | 0.50 |
| Guar hydroxypropyltrimonium chloride | 0.20 |
| Sodium laureth, glycol distearate and cocoamide DEA | 2.00 |
| Polyquaternium-39 (10%) in water | 2.50 |
| Perfume | 0.40 |
| PH adjuster | qs |
| Preservatives | qs |
| Acerola cherry extract | 0.15 |
| Sodium ascorbyl phosphate | 1.5 |
| Purified water pH | to 100 |

The conditioning shampoo is prepared by the same method used to prepare formulation example 1.

### Formulation Example 19 - Hair Mask with Active Mud

| Component | %w/v |
|---|---|
| Molecular sieve | 35.00 |
| Hydrogen vegetable protein | 7.00 |
| Cetearyl alcohol and PEG-23 stearate | 5.00 |
| Cetyl alcohol | 3.00 |
| Cocamide MEA | 3.00 |
| Stearamidopropyl dimethylamine | 3.00 |
| Polyquaternium-39 (10%) in water | 1.00 |
| Fragrance | 0.50 |
| Propylene glycol | to 100 |
| Acerola cherry extract | 0.3 |

The hair mask is prepared as follows. The cetearyl alcohol and PEG-23 stearate mixture, the cetyl alcohol and the propylene glycol are heated together to 70C. This mixture is mixed until homogenous and the rest of the ingredients added and homogenously mixed together. The composition is cooled whilst stirring.

### Formulation Example 20 - Hair Serum

| Component | %w/v |
|---|---|
| Alcohol, t-butyl alcohol and denatonium benzoate | 25.00 |
| Polyquaternium-39 (10%) in water | 1.00 |
| Carbomer 940 | 0.60 |
| Fragrance solubiliser | Qs |
| Fragrance | 0.50 |
| Tetrasodium EDTA | 0.04 |
| 2-Bromo-2-nitropropane-1,3-diol | Qs |
| PH Adjuster | Qs |
| Acerola cherry extract | 0.15 |
| Purified water BP | to 100 |

The hair serum is prepared as follows: The carbomer 940 is dispersed and hydrated in half of the water. The pH of 30% of the water is adjusted to about pH 5.2 and the acerola cherry extract is added. This acerola cherry extract mixture is added to a high shear mixer and mixed until uniform and is then added to the carbomer mixture. The fragrance solubiliser is warmed and mixed with the fragrance and this mixture is added to the bulk acerola cherry extract / carbomer mixture. When the resultant mixture is thoroughly mixed the remaining ingredients are added (the 2-bromo-2-nitropropane-1,3-diol is previously dissolved in purified water before adding to the mixture at this stage). The serum is made up to the required volume with purified water.

### Formulation Example 21 - Hair Rebuilder

| Component | %w/v |
|---|---|
| Guar hydroxypropyltrimonium chloride | 0.30 |
| Alcohol, t-butyl alcohol and denatonium benzoate | 25.00 |
| Fragrance | 0.50 |
| Fragrance solubiliser | Qs |
| Polyquatemium-39 (10%) in water | 1.00 |
| Panthenol and water | 1.00 |
| PH Adjuster | Qs |
| Acerola cherry extract | 0.1 |
| Purified water BP | to 100 |

The hair rebuilder is prepared as follows. The guar hydroxypropyltrimonium chloride is dispersed in 20% of the purified water. The pH of 30% of the water is adjusted to about pH 5.2 and the acerola cherry extract is added. This acerola cherry extract mixture is then mixed in a high shear mixer until uniform and is then added to the guar hydroxypropyltrimonium chloride mixture. The fragrance solubilser is warmed and mixed with the fragrance. This mixture is added to the bulk acerola cherry extract mixture and the resulting mixture is stirred together until well mixed. The remaining ingredients are added and the rebuilder is made up to the required volume with purified water.

### Formulation Example 22 - Shampoo

| Component | %w/v |
|---|---|
| Sodium laureth sulphate and Water | 14.00 |
| Cocamide DEA and DEA-cocoate | 1.50 |
| Cocamidopropyl betaine and salt | 8.00 |
| Lauramide DEA | 1.50 |
| Polyquaternium-39 (10%) in water | 1.00 |
| Panthenol and water | 1.00 |
| Fragrance | 0.50 |
| Preservative | qs |
| Fragrance solubiliser | 0.50 |
| Fatty acid partial glyceride polyglycol ether and ester | 4.00 |
| PH adjuster | qs |
| Viscosity adjuster | qs |
| Acerola cherry extract | 0.1 |
| Ascorbic acid | 0.5 |
| Purified water BP | to 100 |

The shampoo is prepared by the same method used to prepare formulation example 1.

### Formulation Example 23 - Conditioner

| Component | %w/v |
|---|---|
| Polyquaternium-39 (10%) in water | 1.00 |
| Hydrolysed vegetable protein | 7.00 |
| Stearamidopropyl dimethylamine | 0.50 |
| Propylene glycol | 0.50 |
| Dicetyldimonium chloride, ethanol and water | 3.00 |
| Stearyl alcohol and ceteareth-20 | 1.00 |
| Cetyl alcohol | 3.50 |
| Potassium chloride | 0.30 |
| Cyclomethicone | 1.80 |
| Perfume | 0.30 |
| 2-Bromo-2-nitropropane-1,3-diol | qs |
| Viscosity adjuster | qs |
| PH adjuster | qs |
| Acerola cherry extract | 0.1 |
| Ascorbic acid | 0.3 |
| Purified water BP | to 100 |

The conditioner is prepared as follows. The acerola cherry extract and ascorbic acid are pre-dissolved in water and added to 80% of the water. The solution is mixed in a high shear mixer until uniform, and then heated to 80C. The cetyl alcohol and the stearyl alcohol and ceteareth-20 mix are heated to 80C and mixed together with the water phase using the high shear mixer. This mixture is cooled whilst being stirred and when the mixture temperature fell below 30C, the remaining ingredients are added. The product is made up to the required volume with purified water.

### Formulation Example 24 - Leave-in Conditioner

| Component | %w/v |
|---|---|
| Guar hydroxypropyltrimonium chloride | 0.30 |
| Alcohol, t-butyl alcohol and denatomium benzoate | 25.00 |
| Fragrance solubiliser | qs |
| Fragrance | 0.50 |
| Polyquaternium-39 (10%) in water | 1.00 |
| PH adjuster | qs |
| Acerola cherry extract | 0.1 |
| Purified water BP | to 100 |

The conditioner is prepared as follows. The pH of 40% of the water is adjusted to between pH 3.0 and 3.5 and this pH adjusted water is then heated to between 60C and 70C. The acerola cherry extract is added to this heated water whilst being stirred rapidly and the stirring continued until the solution is clear. A further 40% of the water is added to this solution, followed by the guar hydroxypropyltrimonium chloride whilst the water is stirred rapidly for 15 minutes or until the solution became clear. The solution is cooled to below 30C and the remaining ingredients are added.

### Formulation Example 25 - Shampoo

| Component | %w/v |
|---|---|
| Sodium laureth-2 sulphate (27%) | 20.00 |
| Cocoamidopropyl betaine | 4.00 |
| Polyquaternium-7 (8%) in water | 0.20 |
| Perfume | 0.40 |
| Viscosity adjuster | qs |
| PH adjuster | qs |
| Preservative | qs |
| Acerola cherry extract | 0.15 |
| Ascorbyl palmitate | 0.30 |
| Purified Water BP | to 100 |

The shampoo is prepared by the method used to prepare formulation example 1.

### Formulation Example 26 - Hair Gel

| Component | %w/v |
|---|---|
| Purified water BP | to 100 |
| Polyvinylpropionate | 3.50 |
| Carbomer | 1.00 |
| Aminomethyl propanol | 0.40 |
| Dimethicone copolyol | 0.20 |
| PPG-2 Ceteareth-9 | 0.20 |
| Panthenol | 0.08 |
| Disodium EDTA | 0.05 |
| Quaternium-15 | 0.05 |
| Polyquaternium-7 (8%) in water | 0.50 |
| Benzophenone-4 | 0.02 |
| Sodium ascorbate | 0.15 |
| Acerola cherry extract | 0.05 |

The hair gel is prepared by the method used to prepare formulation example 9.

### Formulation Example 27- Conditioner

| Component | %w/v |
|---|---|
| | 0.50 |
| Polyquaternium-7 (8%) in water | |
| Stearamidopropyl dimethylamine | 0.50 |
| Propylene glycol | 0.50 |
| Dicetyldimonium chloride, and ethanol and water | 3.00 |
| Stearyl alcohol and ceteareth-20 | 1,00 |
| Cetyl alcohol | 3.25 |
| Potassium chloride | 0.30 |
| Cyclomethicone | 1.80 |
| Perfume | 0.30 |
| Preservative | qs |
| PH adjuster | qs |
| Ascorbyl palmitate | 0.2 |
| Acerola cherry extract | 0.1 |
| Purified water BP | to 100 |

The conditioner is prepared by the same method used to prepare formulation Example 23.

### Formulation Example 28 - Leave-in Conditioners

| Component | %w/v |
|---|---|
| Panthenol (75%) in water | 0.90 |
| Denatured ethanol | 25.33 |
| Polyquaternium 39 (10%) in water | 0.75 |
| Octyl dimethyl p-aminobenzoate acetate | 0.10 |
| Propylene glycol | 0.86 |
| PEG-40 hydrogenated castor oil | 0.25 |
| 2-Bromo-2-nitropropane-1,3-diol | 0.02 |
| Acerola cherry extract | 0.05 |
| Purified water BP | to 100 |

The leave-in conditioner is prepared as follows. The pH of 40% of the water is adjusted to between 3.0 and 3.5, and this pH adjusted water is heated to between 60°C and 70°C. The acerola cherry extract is added to the heated stirred water, which is stirred until the mixture became clear. A further 40% of the water is added and the solution is cooled to below 35°C when the remaining ingredients are added.

### Formulation Example 29 - Conditioner

| Component | %w/v |
|---|---|
| Cetyl alcohol | 4.00 |
| Cetostearyl alcohol and PEG-20 stearate | 2.00 |
| Hydroxyethylcellulose | 1.00 |
| Polyquaternium-39 (10%) in water | 1.00 |
| D-panthenol (75%) | 0.05 |
| 2-Bromo-2-nitropropane-1,3-diol | 0.02 |
| Methylchloroisothiazolinone, ethylisothiazolinone, magnesium nitrate and water | 0.05 |
| Citric acid | 0.02 |
| Perfume | 0.30 |
| Acerola cherry extract | 0.1 |
| Sodium ascorbyl phosphate | 0.2 |
| Purified water BP | to 100 |

The conditioner is prepared as follows. The hydroxyethylcellulose is hydrated in 80% of the water. The acerola cherry extract and sodium ascorbyl phosphate are pre-dissolved in water and added to the hydroxyethylcellulose mixture which is then mixed in a high shear mixer until uniform and then heated to 80°C. The cetyl alcohol and the cetostearyl alcohol and PEG-20 stearate mix are heated to 80°C and mixed together with the cellulose mixture using the high shear mixer. The resulting mixture is cooled whilst being stirred and when the mixture temperature fell below 30°C the remaining ingredients are added. The product is made up to the required volume with purified water.

### Formulation Example 30 - Colour Corrector

| Component | w/w% |
|---|---|
| Acerola cherry extract. | 0.15 |
| Matlodextrin | 99.85 |

### Method

Maltodextrin is added to the ribbon blender and the mixer set in motion. Acerola cherry extract is added to the moving maltodextrin and mixed until homogenous. Product is packed into air and water tight containers. It is mixed with water prior to use.

### Formulation Example 31 -Colour Corrector 2

| Component | w/w% |
|---|---|
| Acerola cherry extract | 0.15 |
| Sodium Ascorbate | 2.0 |
| Maltodextrin | 97.85 |

### Method

Maltodextrin is added to the ribbon blender and the mixer set in motion. Acerola cherry extract and ascorbate are added to the moving maltodextrin and mixed until homogenous. Product is packed into air and water tight containers. It is mixed with water prior to use.

### Formulation Example 32 - Colour Corrector 3

| Component | w/w% |
|---|---|
| Acerola cherry extract | 0.15 |
| Ascorbic Acid | 93.0 |
| Sodium Ascorbate | 2.0 |
| Maltodextrin | 4.85 |

### Method

Ascorbic acid and maltodextrin are added to the ribbon blender and the mixer set in motion. Acerola cherry extract and sodium ascorbate are added and mixed until homogenous. Product is packed into air and water tight containers. It is mixed with water prior to use.

### Formulation Example 33 - Shine and Conditioner 1

| Component | w/w% |
|---|---|
| Acerola cherry extract | 0.1 |
| Sodium Ascorbate | 1.5 |
| Maltodextrin | 98.4 |

### Method

Maltodextrin is added to the ribbon blender and the blender in motion. Acerola cherry extract and sodium acorbate are added and mixed until homogenous. Product is packed into air and water tight containers. It is mixed with water prior to use.

### Formulation Example 34 - Shine and Conditioner 2

| Component | w/w% |
|---|---|
| Acerola cherry extract | 0.15 |
| Ascorbic Acid | 90.0 |
| Sodium Ascorbate | 2.0 |
| Maltodextrin | 7.85 |

### Method

Ascorbic acid and maltodextrin are added to the ribbon blender and the mixer set in motion. Acerola cherry extract and sodium ascorbate are added and mixed until homogenous. Product is packed into air and water tight containers. It is mixed with water prior to use.

### Assessment of Colour Removal

### Method

Standard dyed hair swatches were soaked in 50 ml of test solutions in a sealed container. The container was then placed in a rotary spinner for 2 minutes. The liquid was then squeezed out of the hair swatch and was analysed for removed hair colour on a UV/Vis spectrophotometer.

The test was repeated 5 times for each test solution. The results were as follows.

Samples tested:
A 5% w/w ascorbic acid
B 0.15% w/w acerola cherry extract
C 4.85% w/w ascorbic acid and 0.15% acerola cherry extract.

All test solutions were prepared in deionised water.

### Results

| Test solution | Mean Absorbance |
|---|---|
| A | 0.226 |
| B | 0.294 |
| C | 0.309 |

The higher the absorbance the more dye that has been removed.

The tests show that 0.15% acerola cherry extract significantly removed more dye than 5.0% ascorbic acid. 0.15% Acerola cherry extract and 4.85% ascorbic acid significantly removed more dye than 5% ascorbic acid. 0.15% Acerola cherry extract and 4.85% ascorbic acid produced a slight improvement in dye removal than 0.15% acerola cherry extract.

Without being bound by any theory, the applicants believe that the acerola cherry extract may have three activities which may explain its activity, namely a pH lowering effect, an antioxidant effect and/or a mild reducing effect. To investigate whether any of these effects were responsible for the hair colour removing properties observed with the hair care compositions of the present invention, the colour removal test was repeated using citric acid or 1M hydrochloric acid as examples of pH-reducing agents, rosemary extract and origanum as examples of antioxidant agents and dithiothreitol as an example of a mild reducing agent. In all experiments, the above materials were present at 5% w/w. The results obtained were:

| | |
|---|---|
| Citric Acid | 0.073 |
| NCl | 0.054 |
| Rosemary | 0.011 |
| Origanum | 0.008 |
| Dithiothreitol | 0.092 |

Thus, these materials show only a small amount of hair colour removing activity when compared to that shown by compositions containing acerola cherry extract. Though the spectrophotometric results indicate that some hair colour had been removed, no loss of colour in the hair swatches was apparent on visual inspection by an experienced observer. In the experiment with dithiothreitol considerable damage was seen to the hair sample. The excellent results shown by compositions containing acerola cherry extract are therefore surprising.

## Claims

1. A method for removing hair colour and/or imparting shine and condition to the hair comprising the steps of
a) providing acerola cherry extract in dry powder form;
b) dissolving said dry powder form in water optionally with other additives to form a haircare composition comprising from 0.01 to 15% w/w acerola cherry extract; and
c) applying said composition to the hair.

2. A method as claimed in claim 1 wherein the composition further comprises additional ascorbic acid or a derivative thereof.

3. A method as claimed in claim 2 wherein the ascorbic acid derivative is one or more of ascorbyl salts, ascorbyl esters, magnesium ascorbyl phosphate, disodium ascorbyl sulphate, ascorbic acid polypeptide and ascorbylmethylsilanol pectinate.

4. A method as claimed in claim 3 in which the ascorbyl salt is selected from sodium ascorbate or magnesium ascorbate and the ascorbyl ester is ascorbyl palmitate.

5. A method as claimed in any preceding claim wherein the composition contains between 0.01 and 3% w/w of the acerola cherry extract.

6. A method as claimed in any one of claims 2 to 5 wherein the composition contains between 0.1 and 20.0% w/w of the additional ascorbic acid or derivative thereof.

7. A method as claimed in any preceding claim wherein the composition contains one or more of preservatives, thickeners, viscosity modifying agent, sequestering agents, resins, slip aids, vitamins, waxes, cosmetically acceptable diluents, cosmetically acceptable carriers, propellants, pearlising agents, perfumes, pH adjusting agents, buffer systems, antidandruff agents and surfactants.

8. A method for removing hair colour from hair to which a hair dye has been previously applied, which method comprises applying to the hair a haircare composition comprising acerola cherry extract.

9. A method as claimed in claim 8 wherein the composition contains additional ascorbic acid or a derivative thereof.

10. A method as claimed in claim 9 wherein the ascorbic acid derivative is one or more of ascorbyl salts, ascorbyl esters, magnesium ascorbyl phosphate, disodium ascorbyl sulphate, ascorbic acid polypeptide and ascorbylmethylsilanol pectinate.

11. A method as claimed in claim 10 in which the ascorbyl salt is selected from sodium ascorbate or magnesium ascorbate and the ascorbyl ester is ascorbyl palmitate.

12. A method as claimed in any of claims 8 to 11 wherein the composition contains between 0.01 and 15% w/w of the acerola cherry extract.

13. A method as claimed in any of claims 8 to 12 wherein the composition contains between 0.01 and 3% w/w of acerola cherry extract.

14. A method as claimed in any of claims 9 to 13 wherein the composition contains between 0.1 and 20.0% w/w of the additional ascorbic acid or derivative thereof.

15. A method as claimed in any of claims 8 to 14 wherein the composition contains one or more of preservatives, thickeners, viscosity modifying agent, sequestering agents, resins, slip aids, vitamins, waxes, cosmetically acceptable diluents, cosmetically acceptable carriers, propellants, pearlising agents, perfumes, pH adjusting agents, buffer systems, antidandruff agents and surfactants.

16. A method as claimed in any of claims 8 to 15 wherein the composition is in the form of a dry powder capable of being dissolved in water.

17. A method as claimed in any one of claims 8 to16, which method comprises dissolving said haircare composition in water to form a solution, and applying the solution to the hair.

18. A method as claimed in claim 17 wherein the solution contains between 0.01 and 3% w/w of acerola cherry extract.

## Patentansprüche

1. Ein Verfahren zur Entfernung von Haarfärbung und/oder um dem Haar Glanz und Stand zu verleihen, das die folgenden Schritte umfasst:
a) Bereitstellung von Acerolakirschextrakt in Trockenpulverform;
b) Auflösung dieser Trockenpulverform in Wasser, optional mit anderen Additiven, um eine Haarpflegeverbindung zu bilden, die zwischen 0,01 und 15 Gew.-% Acerolakirschextrakt enthält; und
c) Anwendung dieser Verbindung im Haar.

2. Ein Verfahren nach Anspruch 1, wobei die Verbindung außerdem zusätzliche Ascorbinsäure oder ein Derivat davon umfasst.

3. Ein Verfahren nach Anspruch 2, wobei das Ascorbinsäure-Derivat aus einem oder mehreren Ascorbyl-Salzen, Ascorbyl-Estern, Magnesium-Ascorbyl-Phosphaten, Dinatrium-Ascorbyl-Sulphaten, Ascorbinsäure-Polypeptiden und Ascorbylmethylsilanol-Pectinaten besteht.

4. Ein Verfahren nach Anspruch 3, bei der das Ascorbyl-Salz aus Natriumascorbat oder Magnesiumascorbat ausgewählt ist und der Ascorbyl-Ester Ascorbyl-Palmitat ist.

5. Ein Verfahren nach einem der vorhergehenden Ansprüche, wobei die Verbindung zwischen 0,01 und 3 Gew.-% Acerolakirschextrakt enthält.

6. Ein Verfahren nach einem der Ansprüche 2 bis 5, wobei die Verbindung zwischen 0,1 und 20,0 Gew.-% zusätzliche Ascorbinsäure oder des Derivates davon enthält.

7. Ein Verfahren nach einem der vorhergehenden Ansprüche, wobei die Verbindung ein oder mehrere Konservierungsmittel, Verdickungsmittel, Wirkstoffe zur Modifizierung der Viskosität, Maskierungsmittel, Harze, Gleitmittel, Vitamine, Wachse, kosmetisch geeignete Verdünner, kosmetisch geeignete Trägerstoffe, Treibmittel, perlende Wirkstoffe, Duftstoffe, Wirkstoffe zur Anpassung des pH-Wertes, Puffersysteme, Antischuppen-Wirkstoffe und Tenside enthält.

8. Ein Verfahren zur Entfernung von Haarfärbung aus dem Haar, das vorher mit einem Haarfärbemittel behandelt wurde, wobei das Verfahren die Anwendung eines Haarpflegemittels umfasst, das Acerolakirschextrakt umfasst.

9. Ein Verfahren nach Anspruch 8, wobei die Verbindung außerdem zusätzliche Ascorbinsäure oder ein Derivat davon enthält.

10. Ein Verfahren nach Anspruch 9, wobei das Ascorbinsäure-Derivat aus einem oder mehreren Ascorbyl-Salzen, Ascorbyl-Estern, Magnesium-Ascorbyl-Phosphaten, Dinatrium-Ascorbyl-Suiphaten, Ascorbinsäure-Polypeptiden und Ascorbylmethylsilanol-Pectinaten besteht.

11. Ein Verfahren nach Anspruch 10, bei der das Ascorbyl-Salz aus Natriumascorbat oder Magnesiumascorbat ausgewählt ist und der Ascorbyl-Ester Ascorbyl-Palmitat ist.

12. Ein Verfahren nach einem der Ansprüche 8 bis 11, wobei die Verbindung zwischen 0,01 und 15 Gew.-% Acerolakirschextrakt enthält.

13. Ein Verfahren nach einem der Ansprüche 8 bis 12, wobei die Verbindung zwischen 0,01 und 3 Gew.-% Acerolakirschextrakt enthält.

14. Ein Verfahren nach einem der Ansprüche 9 bis 13, wobei die Verbindung zwischen 0,1 und 20,0 Gew.-% zusätzliche Ascorbinsäure oder des Derivates davon enthält.

15. Ein Verfahren nach einem der Ansprüche 8 bis 14, wobei die Verbindung ein oder mehrere Konservierungsmittel, Verdickungsmittel, Wirkstoffe zur Modifizierung der Viskosität, Maskierungsmittel, Harze, Gleitmittel, Vitamine, Wachse, kosmetisch geeignete Verdünner, kosmetisch geeignete Trägerstoffe, Treibmittel, perlende Wirkstoffe, Duftstoffe, Wirkstoffe zur Anpassung des pH-Wertes, Puffersysteme, Antischuppen-Wirkstoffe und Tenside enthält.

16. Ein Verfahren nach einem der Ansprüche 8 bis 15, wobei die Verbindung in der Form eines Trockenpulvers vorhanden ist, das in der Lage ist, in Wasser aufgelöst zu werden.

17. Ein Verfahren nach einem der Ansprüche 8 bis 16, wobei das Verfahren die Auflösung dieses Haarpflegemittels in Wasser zur Herstellung einer Lösung und die Anwendung der Lösung auf dem Haar umfasst.

18. Ein Verfahren nach Anspruch 17, wobei die Verbindung zwischen 0,01 und 3 Gew.-% des Acerolakirschextrakt enthält.

## Revendications

1. Un procédé, pour éliminer la couleur des cheveux et/ou rendre brillants et démêler les cheveux, comprenant les étapes consistant à :
a) fournir de l'extrait d'acérola sous la forme de poudre sèche;
b) dissoudre ladite forme de poudre sèche dans de l'eau éventuellement avec d'autres additifs afin d'obtenir une composition pour le soin des cheveux comprenant de 0,01 à 15% en poids d'extrait d'acérola ; et
c) appliquer ladite composition sur les cheveux.

2. Un procédé selon la revendication 1 dans lequel la composition comprend en plus de l'acide ascorbique additionnel ou un dérivé de celui-ci.

3. Un procédé selon la revendication 2 dans lequel le dérivé de l'acide ascorbique est un ou plusieurs dérivés parmi les sels d'ascorbyle, esters d'ascorbyle, l'ascorbyle phosphate de magnésium, l'ascorbyle sulfate de disodium, des polypeptides avec acide ascorbique et le pectinate d'ascorbylméthylsilanol.

4. Un procédé selon la revendication 3 dans lequel le sel d'ascorbyle est choisi entre l'ascorbate de sodium ou l'ascorbate de magnésium et où l'ester d'ascorbyle est le palmitate d'ascorbyle.

5. Un procédé selon quelconque des revendications précédentes dans lequel la composition contient entre 0,01 et 3% en poids d'extrait d'acérola.

6. Un procédé selon l'une quelconque des revendications 2 à 5 dans lequel la composition contient entre 0,1 et 20,0% en poids d'acide ascorbique additionnel ou d'un dérivé de celui-ci.

7. Un procédé selon quelconque des revendications précédentes dans lequel la composition contient un ou plusieurs conservateurs, épaississants, agents modificateurs de la viscosité, agents complexants, résines, coadjuvants de glissement, vitamines, cires, dissolvants cosmétiquement acceptables, véhicules cosmétiquement acceptables, gaz propulseurs, agents formateurs de perles, aromatisants, agents de réglage du pH, systèmes tampon, agents antipellicule et tensioactifs.

8. Un procédé pour décolorer les cheveux auxquels a été préalablement appliquée une coloration capillaire, où le procédé comprend l'application aux cheveux d'une composition pour le soin des cheveux contenant de l'extrait d'acérola.

9. Un procédé selon la revendication 8 dans lequel la composition contient de l'acide ascorbique additionnel ou un dérivé de celui-ci.

10. Un procédé selon la revendication 9 dans lequel le dérivé de l'acide ascorbique est un ou plusieurs dérivés parmi les sels d'ascorbyle, esters d'ascorbyle, l'ascorbyle phosphate de magnésium, l'ascorbyle sulfate de disodium, des polypeptides avec acide ascorbique et le pectinate d'ascorbylméthylsilanol.

11. Un procédé selon la revendication 10 dans lequel le sel d'ascorbyle est choisi entre l'ascorbate de sodium ou l'ascorbate de magnésium et l'ester d'ascorbyle est le palmitate d'ascorbyle.

12. Un procédé selon quelconque des revendications 8 à 11 dans lequel la composition contient entre 0,01 et 15% en poids d'extrait d'acérola.

13. Un procédé selon quelconque des revendications 8 à 12 dans lequel la composition contient entre 0,01 et 3% en poids d'extrait d'acérola.

14. Un procédé selon quelconque des revendications 9 à 13 dans lequel la composition contient entre 0,1 et 20,0% en poids d'acide ascorbique additionnel ou d'un dérivé de celui-ci.

15. Un procédé selon quelconque des revendications 8 à 14 dans lequel la composition contient un ou plusieurs conservateurs, épaississants, agents modificateurs de la viscosité, agents complexants, résines, coadjuvants de glissement, vitamines, cires, dissolvants cosmétiquement acceptables, véhicules cosmétiquement acceptables, gaz propulseurs, agents formateurs de perles, aromatisants, agents de réglage du pH, systèmes tampon, agents antipellicule et tensioactifs.

16. Un procédé selon quelconque des revendications 8 à 15 dans lequel la composition se présente sous la forme d'une poudre sèche pouvant se dissoudre dans l'eau.

17. Un procédé selon quelconque des revendications 8 à 16, dont le procédé comprend la dissolution dans l'eau de ladite composition pour le soin des cheveux afin de former une solution, et l'application de la solution aux cheveux.

18. Un procédé selon la revendication 17 dans lequel la composition contient entre 0,01 et 3% en poids d'extrait d'acérola.
